# EUROPEAN PATENT SPECIFICATION

(11) **EP 1 503 718 B1**
(45) Date of publication and mention of the grant of the patent: **08.07.2009**
(21) Application number: 03723529.8
(22) Date of filing: 09.05.2003
(51) Int. Cl.: A61K 6/10, A61K 6/033

(54) **LOW-DUSTING INVESTMENT COMPOSITION MATERIAL**
LEICHT DISPERGIERBARE EINBETTMASSE
MATERIAU COMPOSITION DE REVETEMENT A FAIBLE EFFUSEMENT

(30) Priority: 10.05.2002 NL 1020577
(43) Date of publication of application: 09.02.2005
(73) Proprietor: Elephant Dental B.V., 1628 PM Hoorn (NL)
(72) Inventor: BETTMAN, Marijke, NL-1625 HZ Hoorn (NL); VAN DER ZEL, Joseph, Maria, NL-1628 TN Hoorn (NL); GRINWIS, Theodorus, Jacobus, NL-1611 JE Bovenkarspel (NL)
(74) Representative: Winckels, Johannes Hubertus F.
(86) International application number: PCT/NL2003/000344
(87) International publication number: WO 2003/099237

(56) References cited:
- EP-A- 0 451 688
- US-A- 3 780 787
- US-A- 4 909 847

## Description

The invention relates to a method for manufacturing a heat-resistant mold for manufacturing a dental restoration, wherein a wax model is manufactured from a powdered heat-resistant material in a conventional manner. The invention further relates to a low-dusting investment material, and in particular to a low-dusting heat-resistant investment material. This material is used in dentistry and especially in dental laboratories for manufacturing molds, and in particular compression and casting molds.

For manufacturing inlays, crowns, bridges, prostheses and other types of dental restoration, in general an impression of the teeth is made by use of specific impression compositions. The thus obtained forms are used to make a model of the teeth, by means of which model the restoration is modeled.

The restoration to be manufactured can be formed by use of, for example, the so-called lost wax method by means of a wax model and heat-resistant investment compositions. For this purpose, the restoration is shaped by means of casting or compression in a heat-resistant investment composition. This technique had long been used for the casting of metal restorations and, for some ten years now, also for compressing glass-ceramic restorations.

As a rule, the heat-resistant investment composition is prepared from a fine-powdered dry mass. A typical composition of this mass is illustrated below in Example 1. This is weighed in a mixing vessel and mixed with water or a special mixing liquid to a slurry. During the processing of the fine-powdered material, and particularly when weighing and filling the mixing vessel, and when mixing with the water or the applied mixing liquid, dusting occurs, sending dust particles into the air, which is undesired for a variety of reasons. In particular, the fine-powdered mass contains respirable silica (particles < 50 µm), of which especially particles smaller than 10 µm are known to be carcinogenic.

The dusting of the fine powder as it occurs during the preparation of the heat-resistant investment composition, needs to be prevented without causing any adverse effects on the further procedure.

The US patent 4,909,847 describes that heat-resistant materials based on quartz, cristobalite, magnesium oxide and a soluble phosphate cause dust formation which makes processing difficult, while health problems can also occur. The dusting problems are solved by adding 0.5-5 wt.% of a wetting agent and 0.01-0.5 wt.% of an anionic surface active agent. Examples of suitable wetting agents are liquid hydrophobic hydrocarbons, liquid hydrophobic fatty acid esters and liquid hydrophobic fatty acids. It is indicated that "since the wetting agent(s) shows a touch of oily nature for reasons of hydrophobic nature and causes a lowering of the efficiency of kneading manipulation, it needs to be used with an anionic surface active agent".

Surface active agents, such as anionic surface active agents, often have the tendency to foam and give rise to adhering residues of investment composition, when these agents are used in the method to which the present invention is directed.

The European patent application 0 451 688 describes investment compositions based on magnesium oxide and phosphate. Isoparaffin is used as a wetting agent. The application of anionic surface active agents is explicitly excluded, because "die Anwesenheit van oberflächenaktiven Substanzen kann jedoch die Einbettmassen nachteilig beeinflussen" ["the presence of surface active agents can, however, adversely affect the investment compositions"]. Although a range of 0.5-5 wt.% isoparaffin is mentioned, a preference is expressed for an amount between 1.5 and 3 wt.%, in which range also the best results are achieved according to the examples in this European patent application.

According to the invention, it has now been found that the dusting problem for the manufacturing method of the invention can be solved by adding to the fine-powdered heat-resistant investment material a hydrophobic liquid which coats the powder particles.

In a first aspect, the invention thus relates to a method for manufacturing a heat-resistant mold for manufacturing a dental restoration, wherein a wax model is manufactured in a conventional manner and wherein from a powdered heat-resistant material consisting of powdered particles coated with a hydrophobic material liquid at room temperature, with water or another mixing liquid, a slurry is prepared, wherein the slurry is applied to the wax model, and wherein the wax model covered in slurry is subjected to a heating step, wherein the wax is burnt off.

In the state of the art, for the prevention of air bubble formation on the wax model when this comes into contact with the investment composition, the wax model is first treated with a surface tension reducing agent, for example a soap or other detergent, to obtain a good wetting. These steps incidentally are generally also carried out in the method according to the invention. In other words, the wax model is first degreased, so that the investment composition slurry can wet the wax model properly without the formation of air bubbles that have an adverse effect on the shape and the surface of the restoration.

It is therefore surprising that with the heat-resistant materials used according to the method according to the invention, hydrophobic liquids are adequate without the wetting of the wax model, which is pre-treated with degreasing materials, being adversely affected.

In a second aspect, the invention relates to a powdered heat-resistant investment composition preferably to be used for application in the method according to the invention, comprising 5-12 wt.% magnesium oxide, 8-15 wt.% phosphate salt, 15-35 wt.% cristobalite, 0.1-0.8 wt.% hydrophobic material liquid at room temperature, and the balance quartz.

Incidentally, it was found that the addition of the hydrophobic material liquid at room temperature had a positive effect on the storage stability of the investment composition. Without wishing to be bound to any theory, it is assumed that the hydrophobic material inhibits some reactions between acids and bases present in the investment composition.

In general, the hydrophobic material is liquid at room temperature and is nonionic.

Very suitable are branched or unbranched hydrocarbons and in particular paraffins and alkanes having at least 9 carbon atoms, such as nonane, decane, undecane, dodecane, tridecane, isoparaffin or vaseline oil, etc.; a commercially available product is for example "paraffin dünnflüssig Ph Eur, BP, NF" ex Merck KgaA, Darmstadt, Germany, which product was also used in the examples below; nonionic liquid polymers and in particular polyols such as polyethylene glycol and polypropylene glycol; alkanolamines such as trialkylamines and in particular triethanolamine and tripropanolamine; siloxanes such as polymethylsiloxane.

Incidentally, C₅-C₁₈ alkyl sulfates such as lauryl sulfate, glycoside butyrates such as sucrose butyrate and glycerol esters are suitable as well.

Also, mixtures of the abovementioned hydrophobic materials can be used.

The hydrophobic material preferably comprises, and most preferably substantially consists of, paraffin oil and in particular paraffin oil with a boiling point between 200 and 500°C, nonamethylundecane, heptamethylnonane, polymethylsiloxane and/or polyethylene glycol.

In particular, isoparaffins are used in pure form or as a mixture. Preferred are 2,2,4,4,6,6,8-heptamethylnonane and 2,2,4,4,6,6,8,8,10-nonamethylundecane. The isoparaffin is obtained in a manner known per se by catalytic oligomerization of isobutane followed by hydrogenation. Isoparaffin has a viscosity in the range from 4 to 8 mPa s/20°C and a boiling point in the range from 210 to 320°C. The flash point is in the range from 90 to 130°C and the ignition temperature from 375 to 420°C.

A very suitable coating material for the powdered heat-resistant investment composition is paraffin oil, for example paraffin oil with a boiling point between 210 and 320°C, such as isoparaffin, or thin liquid paraffin or vaseline oil with a boiling point between 300 and 400°C and a density between 0.87 and 0.89 cm³.

Incidentally, it is known that similar compounds are used with alginate-based impression compositions, which compositions - as indicated above - are used to make impressions of the teeth, which compositions also dust during preparation.

For example, in the Canadian patent 1 161 207, a non-dusting and fast-wetting alginate-based impression material is described, with a coating being applied to the powdered components in an amount of 1-10 wt.% based on the weight of the dry powdered components. This coating is based on natural polymeric dispersants, cellulose ethers and cellulose esters, and synthetic nonionic compounds. In more detail, the application of xanthane gum, hydroxy(m)ethylcellulose, carboxymethylcellulose, polyalginates, alkylene glycols, polyalkylene glycols, triethanolamine, lauryl sulfate, sucrose butyrate and glycerol esters as a coating is described.

In the German "Offenlegungsschrift" 35 35 132 and the US patent 4,695,322, a low-dusting alginate is described. This material is supplied as a dry powder and mixed with water to a creamy substance and used to make an impression of the teeth. In the mouth, the material hardens, after which a positive model can be made by means of gypsum. In order to make the material low-dusting, 2.5-5 wt.% isoparaffin is added.

The US patent 4,543,372, finally, describes a low-dusting alginate-based impression material, to which 1-10 wt.% of a hydrophobic liquid is added, which liquid is a hydrocarbon oil or silicone oil.

Alginate-based impression materials should not adhere to teeth and partly for this reason are made hydrophobic.

In a preferred embodiment of the powdered heat-resistant material according to the invention, the hydrophobic liquid material is present in an amount between 0.1 and 0.8 wt.%. When less than 0.1 wt.% of the hydrophobic liquid is present on the powdered heat-resistant particles, dusting is not sufficiently prevented. Incidentally, to prevent dusting, an amount of at least 0.2 wt.% is generally more effective. When more than 0.8 wt.% of the liquid material is used, no further advantages with regard to the dusting problem are gained, but the wax model becomes greasy and the wetting process is disturbed, as a result of which the mold to be manufactured for, for example, casting dental alloys or compressing a dental ceramic does not lead to the desired dental restorations, for example because the surfaces of the restorations are not sufficiently smooth then.

In an embodiment in which the best results are obtained, the amount of hydrophobic material liquid at room temperature is less than 0.5 wt.% and the preferred range is between 0.2 and 0.5 wt.%. Not only is the dusting of the powdered material prevented, but also, after burning off the wax model, a heat-resistant mold is obtained that provides as smooth a restoration as when no hydrophobic coating material was used.

In general, it has been found practical to add the hydrophobic material to one of the components of the heat-resistant investment composition. Preferably, the hydrophobic material is added to the fraction that gives the most cause for harmful dust formation. However, the hydrophobic material can also be added to the complete mixture or to submixtures.

As already mentioned above, the invention relates to conventional heat-resistant investment material. Typical compositions are described in Examples 1-3. However, all known phosphate-bound investment compositions with fine quartz and/or cristobalite are adequate examples.

The invention is now elucidated with reference to the following, non-limiting examples. Percentages are always related to the weight of the total composition, unless indicated otherwise.

### Example 1 (introductory example)

Three charges of investment composition were manufactured by weighing and mixing the following components:

| | |
|---|---|
| Magnesium oxide | 8.0% |
| (NH₄)H₂PO₄ | 10.0% |
| Quartz | 52.0% |
| Cristobalite | 30.0% |

The powdered material was weighed and placed in a 3-liter plow mixer. The powder was mixed for half an hour.

### Example 2 (introductory example)

In accordance with Example 1, likewise three charges of investment composition were manufactured from the following mixture:

| | |
|---|---|
| Magnesium oxide | 7% |
| NH₄H₂PO₄ | 11% |
| Quartz (SiO₂) | 62% |
| Cristobalite (SiO₂) | 20% |

### Example 3 (introductory example)

In accordance with Examples 1 and 2, likewise three charges of investment composition were manufactured from the following mixture:

| | |
|---|---|
| Magnesium oxide | 9% |
| NH₄H₂PO₄ | 12% |
| Quartz | 47% |
| Cristobalite | 32% |

### Example 4

Before mixing, to the cristobalite fraction of one of the three charges of investment composition powder of example 1, an amount of 0.2 wt.% (based on the dry final weight of the powder) paraffin was added. As paraffin oil, thin liquid paraffin (vaseline oil) with a boiling point between 300 and 400°C and a density of 0.88 was used. Cristobalite and paraffin were mixed together by means of a spatula until no liquid was visible anymore. The resulting lumps were reduced in a 3-liter plow mixer and then mixed with the rest of the components.

### Example 5 and comparative example 6

The same procedure as described in Example 4 was followed, with the difference that to the cristobalite of the second charge, 0.48 (Example 5) and 0.8 wt.% (Example 6) paraffin (related to the dry final weight of the powder) were added instead of 0.2 wt.%.

### Example 7

The investment compositions of the Examples 4-6 and the third charge not treated with hydrophobic material according to Example 1 were tested for relative dust formation. For this purpose, an amount of 50 g powder was put in a glass jar. The jar was closed with a cap and shaken for 30 sec. After shaking, the jar was put down and the cap was removed after 5 sec. In the jar with the untreated investment composition powder, dust formation was clearly visible, and a cloud of dust escaped from the jar when it was opened. In the jars with the investment composition powders treated with paraffin, no dust formation was visible, nor was there any dust formation when they were opened.

To manufacture a mold, each of the heat-resistant powders according to Examples 4-6 was used. Then with the molds test restorations were manufactured. The surface of the test restoration manufactured with the powder of Example 6 was somewhat rougher than those of the other two test restorations.

### Example 8

The dust formation was also tested using the following arrangement. A vacuum bottle was provided with an aerosol filter holder and a vacuum pump. As filter material, a Milipore ® AAWP04700 (0.8 µm, 47 mm diameter) filter was used; this filter was weighed before placing it in the holder. By means of a sprue, 1 gram of a heat-resistant powder mass was inserted in 30 seconds. The bottle was closed and the vacuum pump was turned on for 4 minutes. Then the filter was weighed again.

This procedure was carried out with the composition according to Example 5 and a composition that only differed from Example 5 in the absence of the vaseline oil.

Of the powder to which no vaseline oil had been added, 8.73 wt.% of the inserted amount of powder mass was found to adhere to the filter. Of the powder according to the invention this was found to be only 0.41 wt.%; a reduction by more than a factor of 20.

## Claims

1. A method for manufacturing a heat-resistant mold for manufacturing a dental restoration, wherein a wax model is manufactured in a conventional manner and wherein from a powdered heat-resistant material consisting of powdered particles coated with a hydrophobic material liquid at room temperature, with water or another mixing liquid, a slurry is prepared, wherein the slurry is applied to the wax model, and wherein the wax model covered with slurry is subjected to a heating step, wherein the wax is burnt off, wherein the hydrophobic liquid material is used in the powdered heat-resistant material in an amount of between 0.1 and less than 0.5 wt%.

2. A method according to claim 1, wherein as powdered heat-resistant material, a material is used that is substantially free of anionic surface active agents.

3. A method according to claim 1 or 2, wherein as hydrophobic material a branched or unbranched hydrocarbon is chosen, and in particular a paraffin; an alkane with at least 9 carbon atoms; a polyol; an alkanol amine; a siloxane; a C₈₋₁₈-alkyl sulphate; a glycoside butyrate; a glycerol ester, or a mixture of two or more of the foregoing substances.

4. A method according to any one of the preceding claims, wherein as hydrophobic material, paraffin oil, nonamethylundecane, heptamethylnonane, polymethylsiloxane or polyethylene glycol is chosen.

5. A method according to any one of the preceding claims, wherein as hydrophobic material, a paraffin oil is chosen with a boiling point between 210 and 320°C.

6. A method according to any one of the preceding claims, wherein the hydrophobic liquid material is used in the powdered heat-resistant material in an amount between 0.2 and less than 0.5 wt.%.

7. A powdered heat-resistant investment composition preferably to be used for application in the method according to any one of the preceding claims, comprising 5-12 wt.% magnesium oxide, 8-15 wt.% phosphate salt, 15-35 wt.% cristobalite, 0.1 to less than 0.5 wt.% hydrophobic material liquid at room temperature, and the balance quartz.

8. An investment composition according to claim 7, comprising 0.2 to less than 0.5 wt.% hydrophobic material liquid at room temperature.

9. Investment according to claim 7 or 8, wherein the hydrophobic material liquid at room temperature is isoparaffin

## Patentansprüche

1. Verfahren zur Herstellung einer wärmebeständigen Abgussform zur Herstellung einer Dentalrestoration, wobei ein Wachsmodell in herkömmlicher Weise hergestellt wird und wobei aus einem pulverförmigen wärmebeständigen Material, das aus pulverförmigen Partikeln, beschichtet mit einem bei Raumtemperatur flüssigen hydrophoben Material, besteht, mit Wasser oder einer anderen Mischflüssigkeit eine Aufschlämmung hergestellt wird, wobei die Aufschlämmung auf das Wachsmodell aufgetragen wird und wobei das Wachsmodell, das mit der Aufschlämmung überzogen ist, einem Erwärmungsschritt unterzogen wird, wobei das Wachs abgebrannt wird, wobei das hydrophobe flüssige Material in dem pulverförmigen wärmebeständigen Material in einer Menge von zwischen 0,1 und weniger als 0,5 Gew.-% verwendet wird.

2. Verfahren gemäß Anspruch 1, wobei als pulverförmiges wärmebeständiges Material ein Material verwendet wird, das im Wesentlichen frei von anionischen oberflächenaktiven Mitteln ist.

3. Verfahren gemäß Anspruch 1 oder 2, wobei als hydrophobes Material ein verzweigter oder unverzweigter Kohlenwasserstoff und insbesondere ein Paraffin; ein Alkan mit wenigstens 9 Kohlenstoffatomen; ein Polyol; ein Alkanolamin; ein Siloxan; ein C₆₋₁₈-Alkylsulfat; ein Glycosidbutyrat; ein Glycerinester oder ein Gemisch aus zwei oder mehr der vorstehend genannten Substanzen ausgewählt wird.

4. Verfahren gemäß einem der vorangehenden Ansprüche, wobei als hydrophobes Material Paraffinöl, Nonamethylundecan, Heptamethylnonan, Polymethylsiloxan oder Polyethylenglykol gewählt wird.

5. Verfahren gemäß einem der vorangehenden Ansprüche, wobei als hydrophobes Material ein Paraffinöl mit einem Siedepunkt zwischen 210 und 320°C gewählt wird.

6. Verfahren gemäß einem der vorangehenden Ansprüche, wobei das hydrophobe flüssige Material in dem pulverförmigen wärmebeständigen Material in einer Menge von zwischen 0,2 und weniger als 0,5 Gew.-% verwendet wird.

7. Pulverförmige wärmebeständige Einbettmasse, die vorzugsweise zur Anwendung in dem Verfahren gemäß einem der vorangehenden Ansprüche zu verwenden ist, umfassend 5-12 Gew.-% Magnesiumoxid, 8-15 Gew.-% Phosphatsalz, 15-35 Gew.-% Cristobalit, 0,1 bis weniger als 0,5 Gew.-% bei Raumtemperatur flüssiges hydrophobes Material und als Rest Quarz.

8. Einbettmasse gemäß Anspruch 7, umfassend 0,2 bis weniger als 0,5 Gew.-% bei Raumtemperatur flüssiges hydrophobes Material.

9. Einbettmasse gemäß Anspruch 7 oder 8, wobei das bei Raumtemperatur flüssige hydrophobe Material Isoparaffin ist.

## Revendications

1. Procédé pour fabriquer un moule résistant à la chaleur pour fabriquer une restauration dentaire, dans lequel un modèle en cire est fabriqué de manière conventionnelle et dans lequel on prépare une pâte à partir d'un matériau en poudre résistant à la chaleur constitué de particules en poudre enrobées par un matériau hydrophobe liquide à température ambiante, avec de l'eau ou un autre liquide de mélange, dans lequel la pâte est appliquée au modèle en cire, et dans lequel le modèle en cire recouvert de pâte est soumis à une étape de chauffage, dans laquelle la cire est calcinée, et dans lequel le matériau hydrophobe liquide est utilisé dans le matériau en poudre résistant à la chaleur en une quantité entre 0,1 et moins de 0,5 % en poids.

2. Procédé selon la revendication 1, dans lequel, pour le matériau en poudre résistant à la chaleur, on utilise un matériau qui est essentiellement sans agents tensio-actifs anioniques.

3. Procédé selon la revendication 1 ou 2, dans lequel pour le matériau hydrophobe, on choisit un hydrocarbure ramifié ou non ramifié, et en particulier une parafffine ; un alcane avec au moins 9 atomes de carbone ; un polyol ; une alcanolamine ; un siloxane ; un alkyle sulfate en C₈₋₁₈ ; un glycoside butyrate ; un ester de glycérol, ou un mélange de deux ou plus des substances précédentes.

4. Procédé selon l'une quelconque des revendications précédentes, dans lequel pour le matériau hydrophobe, on choisit de l'huile de paraffine, du nonaméthylundécane, de l'heptaméthylnonane, du polyméthylsiloxane ou du polyéthylène glycol.

5. Procédé selon l'une quelconque des revendications précédentes, dans lequel pour le matériau hydrophobe, on choisit une huile de paraffine avec un point d'ébullition entre 210 et 320°C.

6. Procédé selon l'une quelconque des revendications précédentes, dans lequel le matériau hydrophobe liquide est utilisé dans le matériau en poudre résistant à la chaleur en une quantité entre 0,2 et moins de 0,5 % en poids.

7. Une composition compensatrice en poudre résistant à la chaleur, de préférence pour être utilisée dans l'application du procédé selon l'une quelconque des revendications précédentes, comprenant 5-12% en poids d'oxyde de magnésium, 8-15% en poids de sel de phosphate, 15-35% en poids de cristobalite, de 0,1 à moins de 0,5% en poids de matériau hydrophobe liquide à température ambiante, et le reste de quartz.

8. Composition compensatrice selon la revendication 7, comprenant de 0,2 à moins de 0,5% en poids de matériau hydrophobe liquide à température ambiante.

9. Revêtement selon la revendication 7 ou 8, dans lequel le matériau hydrophobe liquide à la température ambiante est de l'isoparaffine.
